# EUROPÄISCHE PATENTSCHRIFT

(11) **EP 2 695 569 B1**
(45) Veröffentlichungstag und Bekanntmachung des Hinweises auf die Patenterteilung: **13.09.2017**
(21) Anmeldenummer: 13405082.2
(22) Anmeldetag: 19.07.2013
(51) Int. Cl.: A61B 3/00, A61B 3/10, A61B 3/024, A61B 3/032

(54) **AUGENUNTERSUCHUNGSGERÄT**
DEVICE FOR EXAMINING EYES
APPAREIL D'EXAMEN DES YEUX

(30) Priorität: 06.08.2012 CH 12862012
(43) Veröffentlichungstag der Anmeldung: 12.02.2014
(73) Patentinhaber: HAAG-STREIT AG, 3098 Köniz (CH)
(72) Erfinder: NEF, Tobias, 3014 Bern (CH); MÜRI, René, 3360 Herzogenbuchsee (CH); GLOOR, Philipp, 3184 Wünnewil (CH); MOSIMANN, Urs, 3011 Bern (CH)
(74) Vertreter: Stäbler, Roman

(56) Entgegenhaltungen:
- EP-A1- 2 260 753
- WO-A1-99/41641
- DE-A1- 10 350 836
- JP-A- 2008 003 132
- US-A1- 2005 024 722
- US-A1- 2006 227 416
- US-B1- 6 327 020

## Beschreibung

### Technisches Gebiet

Die Erfindung betrifft ein Augenuntersuchungsgerät, insbesondere ein Perimeter, umfassend eine Projektionsvorrichtung und einen konkaven Bildschirm. Weiter betrifft die Erfindung ein Abbildungsverfahren mit einem Augenuntersuchungsgerät.

### Stand der Technik

Unter dem Begriff "Perimetrie" versteht man die systematische Untersuchung, insbesondere die Vermessung des Gesichtsfelds. Damit können äussere und innere Grenzen des Gesichtsfelds bestimmt werden und zudem die Empfindlichkeit des Sehsystems im wahrgenommenen Raum bestimmt werden. Solche Geräte sind weitgehend bekannt. Im Verfahren fixiert der Patient auf einem Bildschirm einen Punkt, während nacheinander optische Reize an verschiedenen Orten des Bildschirms projiziert werden. Anhand der vom Patienten erfassten optischen Reize kann das Gesichtsfeld konstruiert werden.

Die DE 103 50 836 B4 (Frauenhofer) zeigt zum Beispiel einen digitalen Projektor zur Perimetrie sowie einen entsprechenden Perimeter. Mit dem Projektor können digitale Bilder erzeugt werden, welche ein menschliches Blickfeld vollständig oder nahezu vollständig ausfüllen, wobei diese durch digitale Daten vorgebbaren Bilder in weiten Grenzen beliebiger Art und zeitlich veränderlich sein können, ohne dass ein aufwändige mechanische Bildnachführung notwendig wäre. Der Patientenkopf wird dabei neben einer Austrittspupille des Projektors platziert. Die Projektionsfläche ist halbkugelförmig. Die Austrittspupille ist auf Höhe des Kugelzentrums und das zu untersuchende Auge ist seitlich zum Kugelzentrum versetzt angeordnet. Die dabei vom Auge wahrgenommene Verzerrung ist vernachlässigbar. Der Projektor weist drei bildgebende Elemente auf, welche als LCoS-Displays ausgebildet sind.

Die bekannten Augenuntersuchungsgeräte haben den Nachteil, dass sie einen grossen Platzbedarf aufweisen. Ein typisches optisches Projektionssystem, das auch für Augenuntersuchungen benutzt werden kann, zeigt die Schrift US 2006/227416, die als nächstliegenden Stand der Technik angesehen wird und den Gegenstand der Präambel von Anspruch 1 vorwegnimmt.

### Darstellung der Erfindung

Aufgabe der Erfindung ist es, ein dem eingangs genannten technischen Gebiet zugehörendes Augenuntersuchungsgerät zu schaffen, welches besonders kompakt ausgebildet ist.

Die Lösung der Aufgabe ist durch die Merkmale des Anspruchs 1 definiert. Gemäss der Erfindung umfasst das Augenuntersuchungsgerät weiter einen konvexen Reflektor, wobei ein Bild von der Projektionsvorrichtung auf den konvexen Reflektor projizierbar und vom konvexen Reflektor auf den konkaven Bildschirm reflektierbar ist.

Dadurch, dass ein konvexer Reflektor eingesetzt ist, kann das Augenuntersuchungsgerät besonders kompakt ausgebildet sein. Das Augenuntersuchungsgerät ist zudem kostengünstig herstellbar und einfach zu bedienen. Vorzugsweise wird wenigstens ein Teil des von der Projektionsvorrichtung projizierten Lichtes durch den Reflektor reflektiert. Der prozentuale Anteil des von der Projektionsvorrichtung projizierten Lichtes, welches durch den Reflektor reflektiert wird, ist vorzugsweise hoch, insbesondere über 50 %, besonders bevorzugt über 80 %.

Der konkave Bildschirm, auch Cupola genannt, ist vorzugsweise derart geformt, dass das Blickfeld in einem horizontalen und vertikalen Winkelbereich von +/- 90° untersucht werden kann.

Die Projektion auf einen konkaven Bildschirm erfordert durch die auftretenden Verzerrungen des Bildes eine Transformation des zu projizierenden Bildes. Diese Umrechnungen sind hinreichend bekannt, zum Beispiel aus dem Buch Bourke P., Spherical mirror: a new approach to hemispherical dome projection (Planetarian, 2005: 34(4), 6-9) für hemisphärische Bildschirme. Aber auch die Umrechnung für anderweitig geformte konkave Bildschirme und konvexe Reflektoren sind dem Fachmann geläufig und können gegebenenfalls auch iterativ erfolgen, indem das projizierte Bild mit dem zu projizierenden Bild nach jeder Anpassung verglichen wird, bis die Qualität den Anforderungen entspricht.

In einem Abbildungsverfahren mit einem solchen Augenuntersuchungsgerät wird von der Projektionsvorrichtung ein Strahl auf den konvexen Reflektor projiziert und der Strahl vom konvexen Reflektor auf den konkaven Bildschirm reflektiert und auf dem konkaven Bildschirm abgebildet. Das originale Bild wird vorerst geeignet transformiert, anschliessend auf den Reflektor projiziert und von diesem in den konkaven Bildschirm reflektiert. Zusammen mit dem Bild können Prüfobjekte wie Punkte oder Kleinbilder in ein Bild, zum Beispiel ein Vogel in eine Landschaft oder dergleichen projiziert werden. Zusätzlich können Störbilder, wie zum Beispiel geometrische Figuren, Dreiecke oder dergleichen eingeblendet werden. Schliesslich können auch die Grössen der Punkte, Kleinbilder und Störbilder sowie der Kontrast unter den Objekten, das Hintergrundbild und das Timing variiert werden. Durch eine geeignete Wahl der einzelnen Parameter (Art der Objekte, Hintergrundbild, Timing, Kontrast etc.) kann ein Schwierigkeitsgrad definiert werden. Der Proband kann einen Taster betätigen, wenn er den Punkt oder das Kleinbild sieht. Anhand der Reaktionszeit und dem Ort des Punktes oder Kleinbilds kann das Gesichtsfeld des Probanden ermittelt werden.

Der Taster (zum Beispiel eine Maus) ist vorzugsweise über einen Computer mit dem Projektor verlinkt. Die gemessene Reaktionszeit setzt sich allerdings aus der Verzögerung des Systems und der echten Reaktionszeit des Probanden zusammen. Daher wird bevorzugt ein Computer eingesetzt, welcher eine geringere Verzögerungszeit aufweist. Alternativ kann die Verzögerungszeit des Systems fortlaufend gemessen werden, indem zum Beispiel ein Lichtpunkt ausserhalb des Gesichtsfelds auf den Bildschirm projiziert, wenn ein Prüfobjekt projiziert ist. Mit einem hochfrequenten Lichtsensor (z.B. mit einer Messfrequenz von 1000 Hz) kann damit die Verzögerungszeit des Computers gemessen und eliminiert werden.

Bevorzugt ist mit der Projektionsvorrichtung ein flächiges, insbesondere den Bildschirm füllendes Bild projizierbar. Mit "flächiges Bild" ist ein Bild gemeint, welches durch die Projektionsvorrichtung statisch oder bewegt als Film auf den Bildschirm projizierbar ist. Das flächige Bild kann damit mindestens zwei Bereiche mit zumindest unterschiedlichem Kontrast oder Wellenlänge (respektive Farbe) aufweisen, so dass zum Beispiel Farbbilder projiziert werden können. Mit dem konvexen Reflektor kann vorzugsweise das von der Projektionsvorrichtung emittierte Licht auf den gesamten Bildschirm projizieren. So können beispielsweise Filme auf den Bildschirm projiziert werden.

In Varianten kann statt des flächigen Bildes auch lediglich ein Bild, welches einen Bildpunkt mit genau einem Kontrast und einer Wellenlänge umfasst, projizierbar sein.

Vorzugsweise weist der konkave Bildschirm die Form einer Innenseite einer Kugelkalotte, insbesondere einer Halbkugel auf. Der konkave Bildschirm ist vorzugsweise derart geformt, dass das Blickfeld in einem horizontalen und vertikalen Winkelbereich von +/- 90° untersucht werden kann. Dies ist zum Beispiel mit einer Halbkugel erreicht. Durch die Wahl einer Halbkugelform kann auch eine Bildumrechnung einfacher erfolgen.

In Varianten können aber auch andere Formen vorgesehen sein, wie zum Beispiel die Form eines Rotationsparaboloids, eine Rotationsellipsoidhälfte oder dergleichen. Die Form kann auch empirisch ermittelt sein und damit nicht einer idealen mathematischen Funktion folgen. Sie muss auch nicht rotationssymmetrisch sein.

Vorzugsweise ist der Bildschirm aber derart geformt, dass bei der Projektion keine Schattenwürfe entstehen können. Das Bild kann alsdann zum Beispiel iterativ angepasst werden, indem das auf den Bildschirm projizierte Bild jeweils mit dem originalen Bild verglichen wird und anschliessend die Parameter angepasst werden.

Bevorzugt weist der konvexe Reflektor die Form einer Aussenseite eines Kugelkalottenabschnitts, insbesondere die Form einer Kugelkalottenhälfte auf. Damit wird wiederum die Umrechung des Bildmaterials vereinfacht.

Die Form des Reflektors kann ebenfalls die Form eines Rotationsparaboloids, eine Rotationsellipsoidhälfte oder dergleichen aufweisen.

Bevorzugt weist die Projektionsvorrichtung eine Projektionsachse auf, welche mit der Kugelkalottenhöhe des Bildschirms einen Winkel zwischen 70° und 110°, vorzugsweise zwischen 80° und 100°, insbesondere 90° einschliesst. Unter dem Begriff Projektionsachse wird eine mittlere Strahlungsrichtung der Projektionsvorrichtung verstanden, zum Beispiel die Richtung einer Vektorsumme der emitierten Strahlen. Dadurch dass die Projektionsachse besagten Winkel mit der Kugelkalottenhöhe einschliesst, kann das Augenuntersuchungsgerät besonders kompakt aufgebaut werden, da die Projektionsvorrichtung zum Beispiel nicht parallel zur Blickrichtung des Patienten, das heisst neben dem Patientenkopf positioniert werden muss. Damit ergibt sich zudem ein grosser Gestaltungsfreiraum für die Anordnung der Projektionsvorrichtung. Eine Gesamtlänge in Richtung der Kugelkalottenhöhe des Bildschirms des Augenuntersuchungsgeräts kann damit minimiert werden. Die Projektionsvorrichtung ist im Randbereich des konkaven Projektionsvorrichtung ist im Randbereich des konkaven Bildschirms angeordnet. Damit wird ein besonders kompaktes Augenuntersuchungsgerät geschaffen. Die Anordnung im Randbereich hat den Vorteil, dass die Projektion durch den Patientenkopf nicht behindert wird, wie bei bekannten Geräten, bei welchen die Projektionsvorrichtung neben dem Patientenkopf angeordnet ist. Bei letzterer wird nämlich jeweils ein Kompromiss eingegangen, da ja eigentlich sowohl die Projektionsvorrichtung als auch der Patientenkopf idealerweise auf der Kugelkalottenhöhe positioniert sein sollten. Der Patient wird durch die Projektionsvorrichtung auch nicht geblendet, womit eine besonders einfache Handhabung erreicht ist.

In Varianten kann die Projektionsvorrichtung auch ausserhalb obig erwähnter Winkel und Winkelbereiche ausgerichtet sein. Zum Beispiel kann eine Öffnung im Bildschirm vorgesehen sein, durch welche das Bild in Richtung des Reflektors projiziert und von diesem zurück auf den Bildschirm reflektiert wird. In diesem Fall läge der Winkel in einem Bereich zwischen 0° und 20°. Weiter kann die Projektionsvorrichtung wie oben erwähnt neben dem Patientenkopf positioniert sein. Es ist aber auch denkbar, dass zwei oder mehr Projektionsvorrichtungen vorgesehen sind, welche jeweils auf einer Hälfte oder mehreren Teilabschnitten des Bildschirms, welche sich auch überlappen können, das Bild projizieren. In diesem Fall können auch stumpfere Winkel im Bereich von mehr als 110° vorgesehen sein. Überlappungen können in Teilabschnitten von Vorteil sein, in welchen das Flächenverhältnis der Projektionsteilfläche auf dem Bildschirm und der entsprechenden Reflexionsteilfläche auf dem Reflektor gross ist und daher die Lichtintensität eher gering ist. Der konvexe Reflektor ist im Randbereich des konkaven Bildschirms angeordnet. Mit dieser Positionierung wird in einfacher Weise eine Projektion eines den Bildschirm füllenden Bildes mit genau einer Projektionsvorrichtung erreicht. Damit wird weitgehend verhindert, dass der Patient die Projektionsvorrichtung während der Augenuntersuchung sieht und möglicherweise abgelenkt wird. Zudem wird eine kompakte Konstruktion des Augenuntersuchungsgeräts erreicht.

Dem Fachmann ist klar, dass in Varianten der konvexe Reflektor auch anderweitig platziert werden kann, insbesondere auch wenn mehrere Projektionsvorrichtungen eingesetzt sind. Die Position des Reflektors hängt auch weitgehend von der Position der Projektionsvorrichtung ab.

Um einen ästhetischen Abschluss zu schaffen ist vorzugsweise eine Kreisringblende als Abschluss des Bildschirms vorgesehen, in welcher der Patient sein Kopf positioniert. Dadurch, dass mit der Kreisringblende Fremdlicht abgeschirmt werden kann, können zudem die Bildkontraste verbessert werden.

Auf die Kreisringblende kann natürlich auch verzichtet werden. Der konvexe Reflektor und die Projektionsvorrichtung sind im Randbereich des konkaven Bildschirms gegenüberliegend angeordnet. Damit wird eine Projektion des Bildes mit symmetrischer Helligkeit ermöglicht. Vorzugsweise ist der konvexe Reflektor in der Verwendung des Augenuntersuchungsgeräts im Bereich des Kinns des Patienten angeordnet. Diese Anordnung ist vorteilhaft, da ein typisches Gesichtsfeld in Relation zum konkaven Bildschirm im oberen Bereich, das heisst bezüglich einer horizontalen Blickrichtung im vertikal oberen Bereich, durch die Augenbrauen beschränkt ist. Typischerweise beträgt der Gesichtsfeldwinkel über der Horizontalen ungefähr 50° und unter der Horizontalen ungefähr 80°, während der Gesichtsfeldwinkel zur Vertikalen jeweils ungefähr 90° beträgt. Daher ist es sinnvoll, die Projektionsvorrichtung, welche das grösste Volumen einnimmt, in diesem Bereich anzuordnen. Anderseits, wenn der konvexe Reflektor grössere Abmessungen als die Projektionsvorrichtung aufweisen würde, so würde der Reflektor bevorzugt in diesem Bereich angeordnet werden. In diesem Fall würde die Projektionsvorrichtung im Bereich des Kinns angeordnet. Grundsätzlich sind auch die weiteren Positionierungsmöglichkeiten denkbar.

Vorzugsweise ist der konvexe Reflektor innerhalb eines vom konkaven Bildschirm aufgespannten Raumes angeordnet. Damit wird ein kompaktes Augenuntersuchungsgerät geschaffen. Wie oben erwähnt kann damit der ungenutzte Raum im oberen Bereich des Bildschirms, also gegenüber einer allfällig platzierten Kinnstütze, genutzt werden.

In Varianten kann der Reflektor auch ausserhalb des vom Bildschirm aufgespannten Raumes, das heisst in Richtung der Kalottenhöhe des Bildschirms vom Bildschirm weg, und damit ausserhalb des Bildschirms angeordnet sein.

Bevorzugt ist auch die Projektionsvorrichtung innerhalb eines vom konkaven Bildschirm aufgespannten Raumes angeordnet. Damit wird wiederum ein kompakter Aufbau des Augenuntersuchungsgeräts erreicht.

In Varianten kann die Projektionsvorrichtung auch ausserhalb oder teilweise ausserhalb des vom konkaven Bildschirm aufgespannten Raumes angeordnet sein. Grundsätzlich reicht es bei der Positionierung der Projektionsvorrichtung aus, wenn damit auf den Reflektor ein Bild projiziert werden kann. Wenn die Projektionsvorrichtung ausserhalb des Bildschirms aber in derselben Kalottenschnittebene des Bildschirms positioniert wird, so kann eine Öffnung im Bildschirm vorgesehen sein, durch welche die Projektion erfolgen kann. Wird die Projektionsvorrichtung in Richtung der Kalottenhöhe des Bildschirms ausserhalb des Bildschirms positioniert, so ist zu beachten, dass der Kopf des Patienten während der Untersuchung nicht im Strahlengang zwischen der Projektionsvorrichtung und dem Reflektor positioniert ist.

Vorzugsweise ist die Projektionsvorrichtung als LED-Projektor oder als Laser-Projektor ausgebildet. Diese Projektoren sind besonders bevorzugt, da sie in kompakter Form verfügbar sind und eine hinreichende Lichtleistung aufweisen.

Insbesondere der Laser-Projektor hat den Vorteil, dass das Bild immer im Fokus ist und damit auf nahezu beliebig geformten Projektionsflächen scharfe Bilder projiziert werden können, insbesondere bei einer Projektion via konvexen Reflektor auf den konkaven Bildschirm. Es ist keine Fokussierung notwendig. Zudem kann ein hoher Kontrast und ein grosser Gamut erreicht werden.

Der LED-Projektor hat den Vorteil, dass eine hohe Lichtstärke erreicht werden kann. Für die Bedienung ist keine besondere Ausbildung notwendig. Es besteht keine direkte Gefahr für das Auge.

Vorzugsweise weist die Projektionsvorrichtung eine Luminanz zwischen 5 und 10 cd/m², insbesondere ungefähr 7.5 cd/m² auf. In Varianten kann die Luminanz natürlich auch höher oder tiefer sein. Der obere und untere Grenzwert hängt wesentlich von der Projektionsfläche und der Lichtausbeute bei der Reflexion am Reflektor ab.

Das Augenuntersuchungsgerät kann weiter eine Kamera aufweisen, mit welcher die Position der Augen überwacht werden kann.

Das Augenuntersuchungsgerät kann weiter auch für Therapiezwecke eingesetzt werden, um das Gesichtsfeld zum Beispiel nach einem Unfall, nach einem Hirnschlag etc. zu trainieren und zu verbessern.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine Frontansicht in Richtung der Kalottenhöhe des Bildschirms eines schematischen Perimeters;
- Fig. 2: ein Schnittbild des Perimeters quer zur Kalottenhöhe des Bildschirms des Perimeters gemäss Figur 1;
- Fig. 3: eine Seitenansicht eines Schnittbildes des Perimeters in Richtung der Kalottenhöhe des Bildschirms.

Grundsätzlich sind in den Figuren gleiche Teile mit gleichen Bezugszeichen versehen.

### Wege zur Ausführung der Erfindung

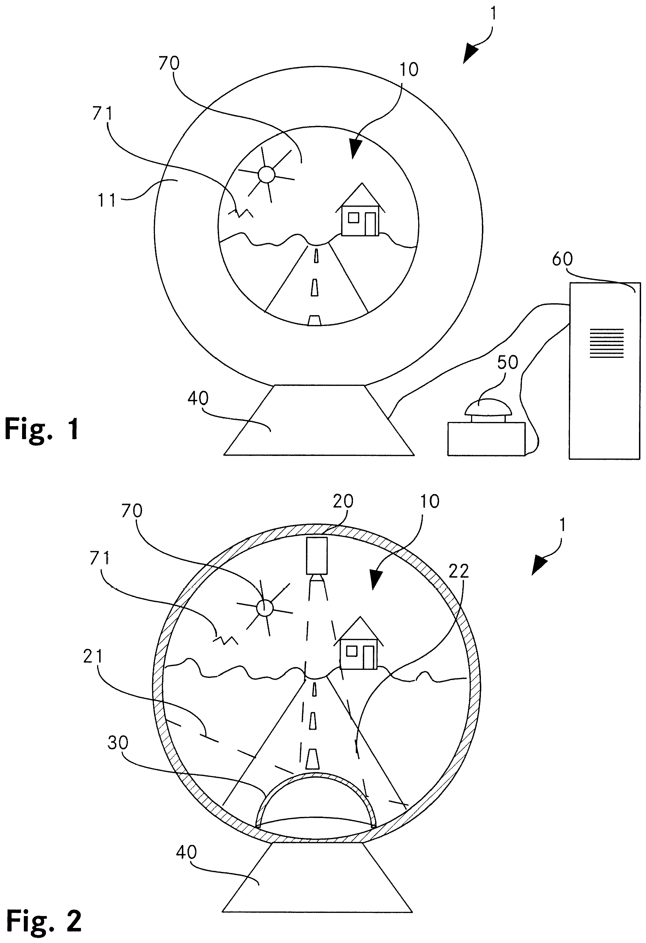

Die Figur 1 zeigt eine Frontansicht in Richtung der Kalottenhöhe des hemisphärischen Bildschirms 10 des schematischen Perimeters 1. Als Basis für den Perimeter 1 kann der Perimeter "Octopus 900" der Haag Streit AG, Köniz (CH) dienen, wobei im Wesentlichen der Projektor und der Reflektor sowie die Datenverarbeitung geändert sind.

Der Perimeter 1 umfasst ein Gehäuse 40, an welchem der hemisphärische Bildschirm 10 montiert ist. Der hemisphärische Bildschirm 10 (nachfolgend Bildschirm 10 genannt) weist einen Durchmesser von ungefähr 60 cm auf. Auf der Vorderseite des Bildschirms 10 ist eine Blende in Form einer Ringscheibenabdeckung 11 angebracht. In der Verwendung positioniert ein Patient oder Proband seinen Kopf innerhalb der Öffnung der Ringscheibenabdeckung 11. Dazu umfasst der Perimeter 1 eine verschiebbare Kinnauflage und einen Anschlag für die Stirn, so dass der Kopf exakt positioniert und ausgerichtet werden kann (nicht dargestellt). Durch die Öffnung der Ringscheibenabdeckung 11 hindurch ist ein auf den Bildschirm 10 projiziertes Bild 70 ersichtlich, welches weiter ein Testobjekt 71 umfasst.

Schliesslich zeigt die Figur 1 einen Taster 50 und einen Computer 60, wobei der Taster 50 mit dem Computer 60 und der Computer 60 mit dem Perimeter 1 verbunden ist.

Im Verfahren wird vorerst der Kopf des Patienten bezüglich der Mitte der beiden Augen zentrisch in der Öffnung der Ringscheibenabdeckung 11 auf der Kinnauflage und dem Stirnanschlag positioniert. Anschliessend fixiert der Patient einen zentralen Punkt (zum Beispiel das Ende der Strasse auf dem Bild 70) und gibt mit der Betätigung des Tasters 50 zu erkennen, wenn er das Testobjekt 71 im projizierten Bild erkannt hat. Mit dem Computer werden die ermittelten Daten ausgewertet, um das Gesichtsfeld des Patienten zu konstruieren.

Die Figur 2 zeigt ein Schnittbild des Perimeters 1 quer zur Kalottenhöhe des hemisphärischen Bildschirms 10 des Perimeters 1 gemäss Figur 1. Im Unterschied zur Figur 1 sind in der Figur 2 nun der Projektor 20 und der Kugelspiegel 30 ersichtlich, welche in der Figur 1 durch die Ringscheibenabdeckung 11 versteckt sind. Der Projektor 20 ist vorliegend als Laserprojektor ausgebildet und in einem vertikal oberen Bereich des Bildschirms 10 angeordnet. Gegenüberliegend, das heisst in einem vertikal unteren Bereich ist der Kugelspiegel 30 angeordnet. Dieser weist die Form einer Viertelkugel auf, wobei gewisse Abweichungen von einer idealen Viertelkugel oder einem 90°-Kugelschnitz bestehen können. Ein vom Projektor emittierter Lichtstrahl 21, 22 wird am Kugelspiegel 30 reflektiert und auf den Bildschirm 10 projiziert. Damit das Bild 70 und das Testobjekt 71 auf dem hemisphärischen Bildschirm 10 nicht verzerrt dargestellt werden, erfolgt vor der Projektion eine geeignete Umrechung oder Transformation der Bilddaten.

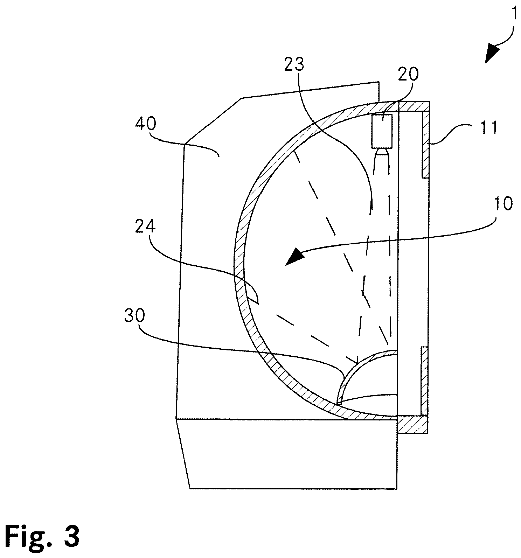

Die Figur 3 zeigt eine Seitenansicht eines Schnittbildes des Perimeters 1 in Richtung der Kalottenhöhe des Bildschirms 10. In dieser Darstellung ist der halbkugelförmige Bildschirm 10 mit der Ringscheibenabdeckung 11 ersichtlich. Wiederum sind zur Illustration zwei Strahlen 23, 24 dargestellt, welche nach der Emission vom Projektor 20 am Kugelspiegel 30 reflektiert und an den Bildschirm projiziert sind.

Zusammenfassend ist festzustellen, dass erfindungsgemäss ein Augenuntersuchungsgerät geschaffen wird, welches kostengünstig in der Herstellung, kompakt und einfach zu bedienen ist.

## Patentansprüche

1. Augenuntersuchungsgerät (1), insbesondere ein Perimeter, umfassend eine Projektionsvorrichtung (20) und einen konkaven Bildschirm (10), wobei das Augenuntersuchungsgerät (1) weiter einen konvexen Reflektor (30) umfasst, wobei ein Bild (70, 71) von der Projektionsvorrichtung (20) auf den konvexen Reflektor (30) projizierbar und vom konvexen Reflektor (30) auf den konkaven Bildschirm (10) reflektierbar ist, **dadurch gekennzeichnet, dass** der konvexe Reflektor (30) und die Projektionsvorrichtung (20) im Randbereich des konkaven Bildschirms (10) gegenüberliegend angeordnet sind.

2. Augenuntersuchungsgerät (1) nach Anspruch 1, **dadurch gekennzeichnet, dass** mit der Projektionsvorrichtung (20) ein flächiges, insbesondere den Bildschirm (10) füllendes Bild (70, 71) projizierbar ist.

3. Augenuntersuchungsgerät (1) nach Anspruch 1 oder 2, **dadurch gekennzeichnet, dass** der konkave Bildschirm (10) die Form einer Innenseite einer Kugelkalotte, insbesondere einer Halbkugel aufweist.

4. Augenuntersuchungsgerät (1) nach einem der Ansprüche 1 bis 3, **dadurch gekennzeichnet, dass** der konvexe Reflektor (30) die Form einer Aussenseite einer Kugelkalottenabschnitts, insbesondere die Form einer Kugelkalottenhälfte aufweist.

5. Augenuntersuchungsgerät (1) nach Anspruch 3 oder 4, **dadurch gekennzeichnet, dass** die Projektionsvorrichtung (20) eine Projektionsachse aufweist, welche mit der Kugelkalottenhöhe des Bildschirms (10) einen Winkel zwischen 70° und 110°, vorzugsweise zwischen 80° und 100°, insbesondere 90° einschliesst.

6. Augenuntersuchungsgerät (1) nach einem der Ansprüche 1 bis 5, **dadurch gekennzeichnet, dass** die Projektionsvorrichtung (20) im Randbereich des konkaven Bildschirms (10) angeordnet ist.

7. Augenuntersuchungsgerät (1) nach einem der Ansprüche 1 bis 6, **dadurch gekennzeichnet, dass** der konvexe Reflektor (30) im Randbereich des konkaven Bildschirms (10) angeordnet ist.

8. Augenuntersuchungsgerät (1) nach einem der Ansprüche 1 bis 7, **dadurch gekennzeichnet, dass** der konvexe Reflektor (30) innerhalb eines vom konkaven Bildschirm (10) aufgespannten Raumes angeordnet ist.

9. Augenuntersuchungsgerät (1) nach einem der Ansprüche 1 bis 8, **dadurch gekennzeichnet, dass** die Projektionsvorrichtung (20) innerhalb eines vom konkaven Bildschirm (10) aufgespannten Raumes angeordnet ist.

10. Augenuntersuchungsgerät (1) nach einem der Ansprüche 1 bis 9, **dadurch gekennzeichnet, dass** die Projektionsvorrichtung (20) als LED-Projektor oder als Laser-Projektor ausgebildet ist.

11. Abbildungsverfahren mit einem Augenuntersuchungsgerät (1) nach einem der Ansprüche 1 bis 10, wobei von der Projektionsvorrichtung (20) ein Strahl auf den konvexen Reflektor (30) projiziert und der Strahl vom konvexen Reflektor (30) auf den konkaven Bildschirm (10) reflektiert und auf dem konkaven Bildschirm (10) abgebildet wird.

## Claims

1. Eye examining instrument (1), more particularly a perimeter, comprising a projection device (20) and a concave screen (10), wherein the eye examining instrument (1) furthermore comprises a convex reflector (30), wherein an image (70, 71) can be projected by the projection device (20) onto the convex reflector (30) and reflected by the convex reflector (30) onto the concave screen (10), **characterized in that** the convex reflector (30) and the projection device (20) are arranged opposite to one another in the edge region of the concave screen (10).

2. Eye examining instrument (1) according to Claim 1, **characterized in that** the projection device (20) can be used to project an areal image (70, 71).

3. Eye examining instrument (1) according to Claim 1, **characterized in that** the concave screen (10) has the shape of an inner side of a spherical cup.

4. Eye examining instrument (1) according to Claim 1, **characterized in that** the convex reflector (30) has the shape of an outer side of a spherical cup section.

5. Eye examining instrument (1) according to Claim 3, **characterized in that** the projection device (20) has a projection axis, which includes an angle of between 70° and 1 10° with the spherical cup height of the screen.

6. Eye examining instrument (1) according to Claim 1, **characterized in that** the projection device (20) is arranged in the edge region of the concave screen (10).

7. Eye examining instrument (1) according to Claim 1, **characterized in that** the convex reflector (30) is arranged in the edge region of the concave screen (10).

8. Eye examining instrument (1) according to Claim 1, **characterized in that** the convex reflector (30) is arranged within a space spanned by the concave screen (10).

9. Eye examining instrument (1) according to Claim 1, **characterized in that** the projection device (20) is arranged within a space spanned by the concave screen (10).

10. Eye examining instrument (1) according to Claim 1, **characterized in that** the projection device (20) is embodied as LED projector or as laser projector.

11. Imaging method using an Eye examining instrument (1) according to Claims 1, wherein the projection device (20) projects a beam onto the convex reflector (30) and the beam is reflected by the convex reflector (30) onto the concave screen (10) and imaged on the concave screen (10).

## Revendications

1. Appareil d'examen oculaire (1), notamment un périmètre ophtalmique, comprenant un dispositif de projection (20) et un écran concave (10), l'appareil d'examen oculaire (1) comprenant en outre un réflecteur convexe (30), une image (70, 71) pouvant être projetée par le dispositif de projection (20) sur le réflecteur convexe (30) et pouvant être réfléchie par le réflecteur convexe (30) sur l'écran concave (10), **caractérisé en ce que** le réflecteur convexe (30) et le dispositif de projection (20) sont disposés opposés l'un à l'autre dans la zone de bordure de l'écran concave (10).

2. Appareil d'examen oculaire (1) selon la revendication 1, **caractérisé en ce que** le dispositif de projection (20) permet de projeter une image (70, 71) plane, notamment qui remplit l'écran (10).

3. Appareil d'examen oculaire (1) selon la revendication 1 ou 2, **caractérisé en ce que** l'écran concave (10) présente la forme d'un côté intérieur d'une calotte sphérique, notamment d'une demi-sphère.

4. Appareil d'examen oculaire (1) selon l'une des revendications 1 à 3, **caractérisé en ce que** le réflecteur convexe (30) présente la forme d'un côté extérieur d'une portion de calotte sphérique, notamment la forme d'une moitié de calotte sphérique.

5. Appareil d'examen oculaire (1) selon la revendication 3 ou 4, **caractérisé en ce que** le dispositif de projection (20) possède un axe de projection qui forme, avec la hauteur de la calotte sphérique de l'écran (10), un angle entre 70° et 110°, de préférence entre 80° et 100°, notamment de 90°.

6. Appareil d'examen oculaire (1) selon l'une des revendications 1 à 5, **caractérisé en ce que** le dispositif de projection (20) est disposé dans la zone de bordure de l'écran concave (10).

7. Appareil d'examen oculaire (1) selon l'une des revendications 1 à 6, **caractérisé en ce que** le réflecteur convexe (30) est disposé dans la zone de bordure de l'écran concave (10).

8. Appareil d'examen oculaire (1) selon l'une des revendications 1 à 7, **caractérisé en ce que** le réflecteur convexe (30) est disposé à l'intérieur d'un espace couvert par l'écran concave (10).

9. Appareil d'examen oculaire (1) selon l'une des revendications 1 à 8, **caractérisé en ce que** le dispositif de projection (20) est disposé à l'intérieur d'un espace couvert par l'écran concave (10).

10. Appareil d'examen oculaire (1) selon l'une des revendications 1 à 9, **caractérisé en ce que** le dispositif de projection (20) est réalisé sous la forme d'un projecteur à LED ou d'un projecteur laser.

11. Procédé de représentation avec un appareil d'examen oculaire (1) selon l'une des revendications 1 à 10, un rayon étant projeté sur le réflecteur convexe (30) par le dispositif de projection (20) et le rayon étant réfléchi par le réflecteur convexe (30) sur l'écran concave (10) et représenté sur l'écran concave (10) .
